(19) Europäisches Patentamt
European Patent Office
Office européen des brevets

(11) Veröffentlichungsnummer: **0 472 118 A1**

(12) **EUROPÄISCHE PATENTANMELDUNG**

(21) Anmeldenummer: **91113709.9**

(51) Int. Cl.5: **C07C 49/84**, C07C 45/61

(22) Anmeldetag: **16.08.91**

(30) Priorität: **24.08.90 DE 4026788**

(43) Veröffentlichungstag der Anmeldung:
**26.02.92 Patentblatt 92/09**

(84) Benannte Vertragsstaaten:
**BE CH DE FR GB IT LI NL**

(71) Anmelder: **BASF Aktiengesellschaft**
**Carl-Bosch-Strasse 38**
**W-6700 Ludwigshafen(DE)**

(72) Erfinder: **Wegner, Guenter, Dr.**
**Paul-Egell-Strasse 7**
**W-6720 Speyer(DE)**
Erfinder: **Karbach, Stefan, Dr.**
**Grundwiesenweg 44**
**W-6730 Neustadt(DE)**
Erfinder: **Smuda, Hubert, dr.**
**Bierhelderweg 7**
**W-6900 Heidelberg(DE)**
Erfinder: **Hickmann, Eckhard, Dr.**
**Kantstrasse 23**
**W-6701 Dannstadt-Schauernheim(DE)**
Erfinder: **Kober, Reiner, Dr.**
**Im Schlittweg 20**
**W-6701 Fussgoenheim(DE)**
Erfinder: **Seele, Rainer, Dr.**
**Leiblstrasse 3**
**W-6701 Fussgoenheim(DE)**
Erfinder: **Zierke, Thomas, Dr.**
**Akazienstrasse 12**
**W-6737 Boehl-Iggelheim(DE)**

(54) **Verfahren zur Herstellung von alpha,alpha-Dialkoxyketonen.**

(57) Verfahren zur Herstellung von α,α-Dialkoxyketonen der allgemeinen Formel I

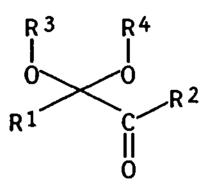

(I),

in der

$R^1$, $R^2$ unabhängig voneinander Wasserstoff, $C_1$- bis $C_{20}$-Alkyl, $C_3$- bis $C_{20}$-Cycloalkyl, $C_4$- bis $C_{30}$-Cycloalkyl-alkyl, $C_9$- bis $C_{30}$-Alkylcycloalkyl, gegebenenfalls durch $C_1$- bis $C_8$-Alkyl, $C_1$- bis $C_8$-Alkoxy, Halogen, $C_1$- bis $C_4$-Halogenalkyl, $C_1$- bis $C_4$-Halogenalkoxy, Phenyl, Phenoxy, Halogenphenyl, Halogenphenoxy und/oder Cyan substituiertes Aryl, $C_7$- bis $C_{20}$-Aralkyl oder Heterocyclus,

$R^2$ zusätzlich

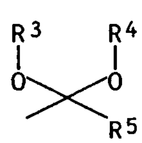

$R^3$, R4 unabhängig voneinander $C_1$- bis $C_{20}$-Alkyl, $C_3$- bis $C_{20}$-Cycloalkyl, Aryl, $C_7$- bis $C_{20}$-Arylalkyl,

EP 0 472 118 A1

gemeinsam eine gegebenenfalls durch $C_1$- bis $C_4$-Alkyl substituierte $C_2$- bis $C_7$-Alkylenkette und

$R^5$ $R^1$ oder gemeinsam mit $R^1$ eine gegebenenfalls durch $C_1$- bis $C_4$-Alkyl substituierte $C_2$- bis $C_7$-Alkylenkette

bedeuten, indem man Ketone bzw. Aldehyde der allgemeinen Formel II

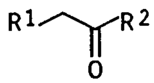

(II)

mit Nitriten der allgemeinen Formel III

$R^{3/4}-(NO_2)_n$ (III)

in der n = 1 oder 2 bedeutet und n = 2 ist, wenn $R^3$ und $R^4$ gemeinsam eine gegebenenfalls durch $C_1$- bis $C_4$-Alkyl substituierte $C_2$- bis $C_7$-Alkylenkette bedeutet, mit der Maßgabe, daß die Nitritgruppen endständig sind oder mit einem Gemisch aus Alkohol der allgemeinen Formel IV

$R^{3/4}-(OH)_n$ IV

in der n = 1 oder 2 bedeutet und n = 2 ist, wenn $R^3$ und $R^4$ gemeinsam eine gegebenenfalls durch $C_1$- bis $C_4$-Alkyl substituierte $C_2$- bis $C_7$-Alkylenkette bedeutet, mit der Maßgabe, daß die Hydroxygruppe endständig ist, und Distickstofftrioxid in einem Molverhältnis von 2:1 in Gegenwart eines sauren Katalysators bei Temperaturen von 0 bis 170 °C, umsetzt.

Die Vorliegende Erfindung betrifft ein verbessertes Verfahren zur HerStellung von $\alpha,\alpha$-Dialkoxyketonen durch Umsetzung von Ketonen oder Aldehyden unter saurer Katalyse mit Alkylnitriten oder einem Gemisch eines Alkohols und Distickstofftrioxid im Molverhältnis 2:1.

Aus der EP-A-222 217 ist die Herstellung von Dialkoxyacetophenonen in Alkoholen als Reaktions- und gleichzeitig Lösungsmittel bekannt. Bei dieser Verfahrensweise waren die Ausbeuten und Produktreinheiten verbesserungswürdig. Besonders wünschenswert war die Vermeidung schwer abtrennbarer Acetalisierungsprodukte der Carbonylfunktion, die bei dieser Fahrweise unvermeidbar auftraten.

Der vorliegenden Erfindung lag daher die Aufgabe zugrunde, den zuvor genannten Nachteilen abzuhelfen.

Demgemäß wurde ein neues und verbessertes Verfahren zur Herstellung von $\alpha,\alpha$-Dialkoxyketonen der allgemeinen Formel I

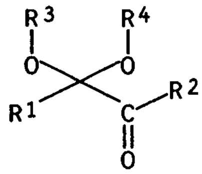

(I),

in der

$R^1$, $R^2$ unabhängig voneinander Wasserstoff, $C_1$- bis $C_{20}$-Alkyl, $C_3$- bis $C_{20}$-Cycloalkyl, $C_4$- bis $C_{30}$-Cycloalkyl-alkyl, $C_9$- bis $C_{30}$-Alkylcycloalkyl, gegebenenfalls durch $C_1$- bis $C_8$-Alkyl, $C_1$- bis $C_8$-Alkoxy, Halogen, $C_1$- bis $C_4$-Halogenalkyl, $C_1$- bis $C_4$-Halogenalkoxy, Phenyl, Phenoxy, Halogenphenyl, Halogenphenoxy und/oder Cyan substituiertes Aryl, $C_7$- bis $C_{20}$-Aralkyl oder Heterocyclus,

$R^2$ zusätzlich

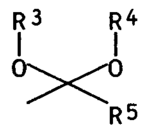

$R^3$, $R^4$ unabhängig voneinander $C_1$- bis $C_{20}$-Alkyl, $C_3$- bis $C_{20}$-Cycloalkyl, Aryl, $C_7$- bis $C_{20}$-Arylalkyl, gemeinsam eine gegebenenfalls durch $C_1$- bis $C_4$-Alkyl substituierte $C_2$- bis $C_7$-Alkylenkette und

$R^5$ $R^1$ oder gemeinsam mit $R^1$ eine gegebenenfalls durch $C_1$- bis $C_4$-Alkyl substituierte $C_2$- bis $C_7$-Alkylenkette

bedeuten, gefunden, welches dadurch gekennzeichnet ist, daß man Ketone bzw. Aldehyde der allgemeinen Formel II

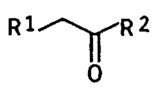

(II)

mit Nitriten der allgemeinen Formel III

$R^{3/4}$-$(NO_2)_n$ (III)

in der n = 1 oder 2 bedeutet und n = 2 ist, wenn $R^3$ und $R^4$ gemeinsam eine gegebenenfalls durch $C_1$- bis $C_4$-Alkyl substituierte $C_2$- bis $C_7$-Alkylenkette bedeutet, mit der Maßgabe, daß die Nitritgruppen endständig sind oder mit einem Gemisch aus Alkohol der allgemeinen Formel IV

$R^{3/4}$-$(OH)_n$ IV

in der n = 1 oder 2 bedeutet und n = 2 ist, wenn $R^3$ und $R^4$ gemeinsam eine gegebenenfalls durch $C_1$- bis $C_4$-Alkyl substituierte $C_2$- bis $C_7$-Alkylenkette bedeutet, mit der Maßgabe, daß die Hydroxygruppe endständig ist, und Distickstofftrioxid in einem Molverhältnis von 2:1 in Gegenwart eines sauren Katalysators bei Temperaturen von 0 bis 170 ° C umsetzt.

Das erfindungsgemäße Verfahren läßt sich wie folgt durchführen:
Die Ketone bzw. Aldehyde der Formel II werden in Gegenwart oder Abwesenheit eines inerten Lösungsmittel mit Alkylnitriten oder einem Gemisch aus einem Alkohol und Distickstofftrioxid im Molverhältnis von 2:1 in Abwesenheit eines Alkoholüberschusses in Gegenwart eines sauren Katalysators bei einer Temperatur von 0 bis 170 ° C, bevorzugt 20 bis 140 ° C, besonders bevorzugt 30 bis 120 ° C umsetzt.

Als Lösungsmittel kommen vorzugsweise Aromaten oder substituierte Aromaten wie Toluol, Xylole, Mesitylen, Chlorbenzol, Fluorbenzol oder Ethylbenzol und aliphatische Verbindungen wie Heptan, Hexan, Cyclohexan, Octan, Isooctan, Petrolether oder Ligroin oder halogenierte aliphatische Verbindungen wie Chloroform, Dichlormethan, Perchlorethylen, Fluorchlormethane, Fluorchlorethane und Gemische dieser Lösungsmittel in Betracht.

Die eingesetzten Alkylnitrite oder Alkohole finden sich als Reste $R^3$ bzw. $R^4$ in den Endprodukten wieder. Vorzugsweise werden Methylnitrit, Ethylnitrit, Isopropylnitrit, n-Propylnitrit, n-Butylnitrit, 2,2-Dimethylpropylen-1,3-dinitrit und Glycoldinitrit bzw. die analogen Alkohole mit Distickstofftrioxid im stöchiometrischen Verhältnis 2:1 pro Hydroxyfunktion eingesetzt.

Als Katalysatoren werden Säuren verwendet. Vorzugsweise werden HCl, HBr, Toluolsulfonsäure, Methansulfonsäure, Schwefelsäure, Phosphorsäure, Essigsäure, Trifluoressigsäure und Tetrafluorborwasserstoffsäure verwendet. Pro mol Keton oder Aldehyd werden 0,05 bis 1 Mol saurer Katalysator zugesetzt.

Als Reaktionstemperatur kann bei 0 bis 170°C gearbeitet werden, vorzugsweise jedoch bei der Siedetemperatur des Lösungsmittels, im allgemeinen jedoch bei 30 bis 90°C. Bei entsprechenden Lösungsmitteln oder bei Normaldruck gasförmigen Reaktionskomponenten kann die Reaktion auch unter Druck durchgeführt werden.

Das erfindungsgemäße Verfahren kann sowohl diskontinuierlich als auch kontinuierlich durchgeführt werden.

Nach beendeter Umsetzung, die im allgemeinen 1 bis 10 Stunden in Anspruch nimmt, wird durch Zugabe von Basen (z.B. Natriumhydroxid, Kaliumhydroxid, Kaliumcarbonat, Calciumoxid oder Alkoholaten ein pH-Bereich von 7 bis 10 eingestellt, wobei auch überschüssiges Nitrit zerstört wird.

Die gewaschene neutralisierte Lösung wird destilliert und durch Fraktionieren die Wertprodukte gewonnen.

Die Substituenten $R^1$, $R^2$, $R^3$, $R^4$ und $R^5$ in den Verbindungen I, II, III und IV haben folgende Bedeutungen:

$R^1$, $R^2$

- unabhängig voneinander
- Wasserstoff
- $C_1$- bis $C_{20}$-Alkyl, bevorzugt $C_1$- bis $C_8$-Alkyl, besonders bevorzugt $C_1$- bis $C_4$-Alkyl wie Methyl, Ethyl, n-Propyl, iso-Propyl, n-Butyl, iso-Butyl, sec.-Butyl und tert.-Butyl,
- $C_3$- bis $C_{20}$-Cycloalkyl, bevorzugt $C_3$- bis $C_8$-Cycloalkyl wie Cyclopropyl, Cyclobutyl, Cyclopentyl, Cyclohexyl, Cycloheptyl und Cyclooctyl, besonders bevorzugt Cyclopentyl und Cyclohexyl,
- $C_4$- bis $C_{30}$-Cycloalkyl-alkyl, bevorzugt $C_4$- bis $C_{20}$-Cycloalkyl-alkyl wie Cyclopentyl-methyl, Cyclohexyl-methyl, 1-Cyclohexyl-ethyl und 2-Cyclohexyl-ethyl,
- $C_4$- bis $C_{30}$-Alkyl-cycloalkyl, bevorzugt $C_4$- bis $C_{20}$-Alkylcycloalkyl wie 4-Methyl-cyclohexyl, 4-Ethyl-cyclohexyl und 2-Methyl-cyclohexyl,
- Aryl, wie Phenyl, 1-Naphthyl und 2-Naphthyl, bevorzugt Phenyl,
- ein- bis dreifach durch $C_1$- bis $C_8$-Alkyl substituiertes Aryl, bevorzugt ein- bis dreifach durch $C_1$- bis $C_4$-Alkyl substituiertes Phenyl, wie 2-Methylphenyl, 4-Methylphenyl, 2-Ethylphenyl, 4-Ethylphenyl, 3-Methylphenyl, 2,4-Dimethylphenyl, 3,4-Dimethylphenyl und 3,4,5-Trimethylphenyl,
- ein- bis dreifach durch $C_1$- bis $C_8$-Alkoxy substituiertes Aryl, bevorzugt ein- bis dreifach durch $C_1$- bis $C_4$-Alkoxy substituiertes Phenyl, wie 2-Methoxyphenyl, 2-Ethoxyphenyl, 3-Methoxyphenyl, 4-Methoxyphenyl, 3-Ethoxyphenyl, 4-Ethoxyphenyl, 3,4-Dimethoxyphenyl und 3,4,5-Trimethoxyphenyl,
- ein- bis dreifach durch $C_1$- bis $C_4$-Halogenalkyl substituiertes Aryl, bevorzugt ein- bis dreifach durch $C_1$- bis $C_2$-Fluor- und Chloralkyl Phenyl, besonders bevorzugt ein- bis dreifach durch Trifluormethyl und Trichlormethyl, substituiertes Phenyl, wie 4-Trifluormethylphenyl und 4-Trichlormethylphenyl,

- ein- bis dreifach durch $C_1$- bis $C_4$-Halogenalkoxy substituiertes Aryl, bevorzugt ein- bis dreifach durch $C_1$- bis $C_2$-Fluor- und Chloralkoxy Phenyl, besonders bevorzugt ein- bis dreifach durch Trifluormethoxy und Trichlormethoxy substituiertes Phenyl, wie Trifluormethoxyphenyl,
- ein- bis dreifach durch Halogen substituiertes Aryl, bevorzugt ein- bis dreifach durch Fluor oder Chlor substituiertes Phenyl, wie 4-Chlorphenyl, 3,4-Dichlorphenyl, 4-Bromphenyl, 4-Fluorphenyl und 4-Fluor-3-chlorphenyl,
- ein- bis dreifach durch Halogenphenyl substituiertes Aryl, bevorzugt ein- bis dreifach durch Fluor- und/oder Chlorphenoxy substituiertes Phenyl wie (4-Chlorphenyl)-phenyl,
- ein- bis dreifach durch Halogenphenoxy substituiertes Aryl, bevorzugt ein- bis dreifach durch Fluor- und/oder Chlorphenoxy substituiertes Phenyl, wie (4-Fluorphenoxy)-phenyl,
- ein- bis dreifach durch Cyano substituiertes Aryl, bevorzugt ein- bis dreifach durch Cyano substituiertes Phenyl, wie 2-Cyanophenyl, 3-Cyanophenyl und 4-Cyanophenyl,
- $C_7$- bis $C_{20}$-Arylalkyl, bevorzugt $C_7$- bis $C_{12}$-Arylalkyl wie Benzyl, Phenylethyl, Phenylpropyl und Phenylisopropyl,
- im Arylteil ein- bis dreifach durch Halogen substituiertes $C_7$- bis $C_{20}$-Aryl-alkyl, bevorzugt im Phenylteil ein- bis dreifach durch Fluor oder Chlor $C_7$- bis $C_{10}$-Phenyl-alkyl, wie 4-Fluorbenzyl, 4-Chlorbenzyl, 2,4-Dichlorbenzyl und 3,4-Dichlorbenzyl,
- im Arylteil ein- bis dreifach durch $C_1$- bis $C_8$-Alkyl substituiertes $C_7$- bis $C_{20}$-Aryl-alkyl, bevorzugt im Phenylteil ein- bis dreifach durch $C_1$- bis $C_4$-Alkyl substituiertes $C_7$- bis $C_{10}$-Phenyl-alkyl, besonders bevorzugt im Phenylteil ein- bis dreifach durch $C_1$- bis $C_2$-Alkyl substituiertes $C_7$- bis $C_{10}$-Phenylalkyl, wie 4-Methylphenyl, 4-Ethylphenyl und 4-Methylphenethyl,
- im Arylteil ein- bis dreifach durch $C_1$- bis $C_8$-Alkoxy substituiertes $C_7$- bis $C_{20}$-Aryl-alkyl, bevorzugt im Phenylteil ein- bis dreifach durch $C_1$- bis $C_4$-Alkoxy $C_7$- bis $C_{10}$-Phenylalkyl, besonders bevorzugt im Phenylteil ein- bis dreifach durch $C_1$- bis $C_2$-Alkoxy substituiertes $C_7$- bis $C_{10}$-Phenylalkyl, wie 4-Methoxybenzyl, 4-Ethoxybenzyl und 4-Methoxyphenethyl,
- im Arylteil ein- bis dreifach durch $C_1$- bis $C_4$-Halogenalkyl, substituiertes $C_7$- bis $C_{20}$-Arylalkyl, bevorzugt im Phenylteil ein- bis dreifach durch $C_1$- bis $C_2$-Fluor- und Chloralkyl substituiertes $C_7$- bis $C_{10}$-Phenylalkyl, besonders bevorzugt im Phenylteil ein- bis dreifach durch Trifluormethyl und Trichlormethyl substituiertes $C_7$-$C_{10}$-Phenylalkyl, wie 4-Trifluormethylbenzyl und 4-Trichlormethylbenzyl,
- im Arylteil ein- bis dreifach durch $C_1$- bis $C_4$-Halogenalkoxy substituiertes $C_7$- bis $C_{20}$-Arylalkyl, bevorzugt im Phenylteil ein- bis dreifach durch $C_1$- bis $C_2$-Halogenalkoxy substituiertes $C_7$- bis $C_{10}$-Phenyl-alkyl, besonders bevorzugt ein- bis dreifach durch Trifluormethoxy und Trichlormethoxy substituiertes $C_7$- bis $C_{10}$-Phenylalkyl, wie 4-Trifluormethoxybenzyl und 4-Trichlormethoxybenzyl,
- ein- bis dreifach durch Halogenphenyl substituiertes $C_7$- bis $C_{20}$-Arylalkyl, bevorzugt ein- bis dreifach durch Fluor- und/oder Chlorphenyl substituiertes $C_7$- bis $C_{12}$-Phenylalkyl, wie 4-Chlorphenylphenethyl und 4-Fluorphenylphenethyl,
- ein- bis dreifach durch Halogenphenoxy substituiertes $C_7$- bis $C_{20}$-Arylalkyl, bevorzugt ein- bis dreifach durch Fluor- und/oder Chlorphenoxy substituiertes $C_7$- bis $C_{12}$-Phenylalkyl, wie 2-Chlorphenoxyphenylmethoxy und 4-Chlorphenoxyphenylmethyl,
- ein- bis dreifach durch Cyano substituiertes $C_7$- $C_{20}$-Arylalkyl, bevorzugt ein- bis dreifach durch Cyano substituiertes $C_7$- bis $C_{12}$-Phenylalkyl, wie 2-Cyanobenzyl, 4-Cyanobenzyl, 2-Cyanophenethyl und 4-Cyanophenethyl,
- durch ein, zwei oder drei Phenylgruppen substituiertes Phenyl, wie 2-(Phenyl)phenyl, 3-(Phenyl)-phenyl, 4-(Phenyl)-phenyl und 3,4-(Diphenyl)-phenyl,
- durch ein, zwei oder drei Phenoxygruppen substituiertes Phenyl, wie 4-Phenoxyphenyl und 2-Phenoxyphenyl,
- durch Halogen und $C_1$- bis $C_4$-Alkyl zwei- oder dreifach substituiertes Phenyl, wie 2-Methyl-4-chlorphenyl und 3-Methyl-4-fluorphenyl,
- durch Halogen und $C_1$- bis $C_4$-Alkoxy zwei- oder dreifach substituiertes Phenyl, wie 3-Chlor-4-methoxyphenyl,
- durch Halogen und $C_1$- bis $C_4$-Halogenalkyl zwei- oder dreifach substituiertes Phenyl, wie 2-Chlor-4-trifluormethylphenyl,

- durch Halogen und Phenoxy zwei- oder dreifach substituiertes Phenyl, wie 3-Chlor-4-phenoxyphenyl,
- durch $C_1$- bis $C_4$-Alkyl und $C_1$- bis $C_4$-Alkoxy zwei- oder dreifach substituiertes Phenyl, wie 2-Methyl-4-methoxyphenyl,
- durch $C_1$- bis $C_4$-Alkyl und $C_1$- bis $C_4$-Halogenalkyl zwei- oder dreifach substituiertes Phenyl, wie 3-Methyl-4-trichlormethylphenyl,
- durch $C_1$- bis $C_4$-Alkyl und Phenoxy zwei- oder dreifach substituiertes Phenyl, wie 2-Methyl-4-phenoxyphenyl,
- durch $C_1$- bis $C_4$-Alkoxy und $C_1$- bis $C_4$-Halogenalkyl zwei- oder dreifach substituiertes Phenyl, wie 3-Trifluormethyl-4-methoxyphenyl,
- durch $C_1$- bis $C_4$-Alkoxy und Phenoxy zwei- oder dreifach substituiertes Phenyl, wie 3-Methoxy-4-phenoxyphenyl,
- durch $C_1$- bis $C_4$-Halogenalkyl und Phenoxy zwei- oder dreifach substituiertes Phenyl, wie 3-Trifluormethyl-4-phenoxyphenyl,
- durch Halogen, $C_1$- bis $C_4$-Alkyl und $C_1$- bis $C_4$-Alkoxy dreifach substituiertes Phenyl, wie 2-Chlor-3-tert.butyl-4-methoxyphenyl,
- durch Halogen, $C_1$- bis $C_4$-Alkyl und $C_1$- bis $C_4$-Halogenalkyl dreifach substituiertes Phenyl, wie 2-Methyl-3-chlor-4-trifluormethylphenyl,
- durch Halogen, $C_1$- bis $C_4$-Alkyl und Phenoxy dreifach substituiertes Phenyl, wie 4-Chlor-2-ethyl-3-phenoxyphenyl,
- durch Halogen, $C_1$- bis $C_4$-Alkoxy und $C_1$- bis $C_4$-Halogenalkyl dreifach substituiertes Phenyl, wie 3-Chlor-4-methoxy-3-trifluormethylphenyl,
- durch Halogen, $C_1$- bis $C_4$-Alkoxy und Phenoxy dreifach substituiertes Phenyl, wie 2-Fluor-4-ethoxy-3-phenoxyphenyl,
- durch Halogen, $C_1$- bis $C_4$-Halogenalkyl und Phenoxy dreifach substituiertes Phenyl, wie 4-Fluor-3-trifluormethyl-2-phenoxyphenyl,
- durch $C_1$- bis $C_4$-Alkyl, $C_1$- bis $C_4$-Alkoxy und $C_1$- bis $C_4$-Halogenalkyl dreifach substituiertes Phenyl, wie 4-Methyl-3-methoxy-2-trichlormethylphenyl,
- durch $C_1$- bis $C_4$-Alkyl, $C_1$- bis $C_4$-Alkoxy und Phenoxy dreifach substituiertes Phenyl, wie 4-Methyl-3-ethoxy-2-phenoxyphenyl,
- durch $C_1$- bis $C_4$-Alkyl, $C_1$- bis $C_4$-Halogenalkyl und Phenoxy dreifach substituiertes Phenyl, wie 2-Methyl-4-trifluormethyl-3-phenoxyphenyl,
- durch $C_1$- bis $C_4$-Alkoxy, $C_1$- bis $C_4$-Halogenalkyl und Phenoxy dreifach substituiertes Phenyl, wie 4-Methoxy-2-trichlormethyl-3-phenoxyphenyl,

$R^2$

- zusätzlich

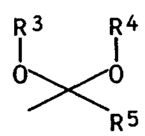

$R^3$, $R^4$

- unabhängig voneinander
- $C_1$- bis $C_{20}$-Alkyl, bevorzugt $C_1$- bis $C_8$-Alkyl, besonders bevorzugt $C_1$- bis $C_4$-Alkyl wie Methyl, Ethyl, n-Propyl, iso-Propyl, n-Butyl, iso-Butyl, sec.-Butyl und tert.-Butyl,
- $C_3$- bis $C_{20}$-Cycloalkyl, bevorzugt $C_3$- bis $C_8$-Cycloalkyl wie Cyclopropyl, Cyclobutyl, Cyclopentyl, Cyclohexyl, Cycloheptyl und Cyclooctyl, besonders bevorzugt Cyclopentyl und Cyclohexyl,
- Aryl wie Phenyl, 1-Naphthyl und 2-Naphthyl, bevorzugt Phenyl,
- $C_7$- bis $C_{20}$-Arylalkyl, bevorzugt $C_7$- bis $C_{12}$-Arylalkyl wie Benzyl, Phenylethyl, Phenylpropyl und Phenylisopropyl,
- gemeinsam eine gegebenenfalls durch $C_1$- bis $C_4$-Alkyl substituierte $C_2$- bis $C_7$-Alkylenkette wie $(CH_2)_2$, $(CH_3)_3$, $(CH_2)_4$, $(CH_2)_5$, $(CH_2)_6$, $(CH_2)_7$, $CH_2$-$CH(CH_3)$, $CH$-$(CH_3)$-$CH(CH_3)$, $CH_2$-$C(CH_3)_2$, $CH_2$-$CH_2$-$CH(CH_3)$, $CH_2$-$CH(CH_3)$-$CH_2$, $CH_2$-$C(CH_3)_2$-$CH_2$, $(CH_2)_3$-$CH(CH_3)$ und $(CH_2)_2$-$CH(CH_3)$-$CH_2$, bevorzugt $(CH_2)_2$, $(CH_2)_3$, $(CH_2)_4$, $(CH_2)_5$, $(CH_2)_6$, $(CH_2)_7$ und $CH_2$-$C(CH_3)_2$

$R^5$

- alle Bedeutungen von $R^1$
- gemeinsam mit $R^1$ eine gegebenenfalls durch $C_1$- bis $C_4$-Alkyl substituierte $C_2$- bis $C_7$-Alkylenkette wie $(CH_2)_2$, $(CH_3)_3$, $(CH_2)_4$, $(CH_2)_5$, $(CH_2)_6$, $(CH_2)_7$, $CH_2$-$C(CH_3)_2$-$CH_2$, $CH_2$-$CH(CH_3)$, $CH(CH_3)$-$CH(CH_3)$, $CH_2$-$C(CH_3)_2$, $CH_2$-$CH_2$-$CH(CH_3)$, $CH_2$-$CH(CH_3)$-$CH_2$, $CH_2$-$C(CH_3)_2$-$CH_2$, $(CH_2)_3$-$CH(CH_3)$ und $(CH_2)_2$-$CH(CH_3)$-$CH_2$, bevorzugt $(CH_2)_2$, $(CH_2)_3$, $(CH_2)_4$, $(CH_2)_5$, $(CH_2)_6$, $(CH_2)_7$ und $CH_2$-$C(CH_3)_2$.

Die nach dem neuen Verfahren herstellbaren α,α-Dialkoxyketone sind wertvolle Zwischenprodukte für die Synthese von Pflanzenschutzmitteln und Pharmazeutika (EP-A 94 564, EP-A 38 196 038, EP-A-196 583, DE-A 30 47 726 und DE-A 31 50 204).

Beispiele

Beispiel 1

Herstellung von α,α-Dimethoxyacetophenon

61 g (0,5 Mol) Acetophenon werden in 750 ml Toluol vorgelegt, 15 g gasförmige HCl eingeleitet und auf 40 bis 45 °C erwärmt. Bei dieser Temperatur werden 1,2 Mol Methylnitrit innerhalb von 3 bis 4 Stunden eingegast. Die Reaktionslösung wird mit 20 %iger Natronlauge alkalisch gestellt und die entstehenden Phasen getrennt und gewaschen. Nach Destillation werden 85 % reines α,α-Dimethoxyacetophenon, erhalten.

Analog Beispiel 1 sind die in der folgenden Tabelle aufgeführten Verbindungen hergestellt worden oder herstellbar.

Tabelle

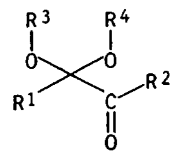

| Bsp. Nr. | $R^1$ | $R^2$ | $R^3$ | $R^4$ | Sdp. | 250 MHz $^1H$-NMR in $CDCl_3$ δ [ppm] | Ausbeute [%] |
|---|---|---|---|---|---|---|---|
| 1 | H | $C_6H_5$ | $CH_3$ | $CH_3$ | | 3,47 (6H,$OCH_3$), 5,22 (1H,CH), 7,45, 7,57, 8,11 (5H,Ar-H) | 85 |
| 2 | H | 2-$C_4H_3S$ | $CH_3$ | $CH_3$ | | 3,49 (6H,$OCH_3$), 5,08 (1H,CH), 7,14, 7,69, 8,02 (3H,Het-H) | 75 |
| 3 | H | 3-$C_4H_3S$ | $CH_3$ | $CH_3$ | | 3,47 (6H,$OCH_3$), 5,03 (1H,CH), 7,30, 7,64, 8,39 (3H,Het-H) | 78 |
| 4 | H | $C_4H_3O$ | $CH_3$ | $CH_3$ | | | |
| 5 | H | 4-Cl-$C_6H_4$ | $CH_3$ | $CH_3$ | | | |
| 6 | H | 4-Br-$C_6H_4$ | $CH_3$ | $CH_3$ | | | |
| 7 | H | 4-F-$C_6H_4$ | $CH_3$ | $CH_3$ | 60-75°C/0,3 mbar | 3,47 (6H,$OCH_3$), 5,14 (1H, CH), 7,11, 8,17 (4H, Ar-H) | 85 |
| 8 | H | 2-F-$C_6H_4$ | $CH_3$ | $CH_3$ | | | |
| 9 | H | 2,4-$Cl_2$-$C_6H_3$ | $CH_3$ | $CH_3$ | | | |
| 10 | H | 2,4-$F_2$-$C_6H_3$ | $CH_3$ | $CH_3$ | | | |
| 11 | H | 4-$CH_3$-$C_6H_3$ | $CH_3$ | $CH_3$ | | | |
| 12 | H | 4-$CF_3$-$C_6H_4$ | $CH_3$ | $CH_3$ | 75-78°C/0,3 mbar | 3,50 (6H,$OCH_3$), 5,12 (1H,CH), 7,11, 8,17 (4H,Ar-H) | 82 |
| 13 | H | 3-$CF_3$-$C_6H_4$ | $CH_3$ | $CH_3$ | | | 78 |
| 14 | H | $(CH_3)_3C$ | $CH_3$ | $CH_3$ | | 1,20 (9H,$CH_3$), 3,39 (6H,$OCH_3$), 4,96 (1H,CH) | |

Tabelle (Fortsetzung)

| Bsp. Nr. | $R^1$ | $R^2$ | $R^3$ | $R^4$ | Sdp. | 250 MHz $^1H$-NMR in $CDCl_3$ δ [ppm] | Ausbeute [%] |
|---|---|---|---|---|---|---|---|
| 15 | H | $C_6H_5$ | $C_5H_{11}$ | $C_5H_{11}$ | | | |
| 16 | H | $4-Cl-C_6H_4$ | $C_5H_{11}$ | $C_5H_{11}$ | | | |
| 17 | H | $4-Br-C_6H_4$ | $C_5H_{11}$ | $C_5H_{11}$ | | | |
| 18 | H | $4-F-C_6H_4$ | $C_5H_{11}$ | $C_5H_{11}$ | | | |
| 19 | H | $2-F-C_6H_4$ | $C_5H_{11}$ | $C_5H_{11}$ | | | |
| 20 | H | $2,4-Cl_2-C_6H_3$ | $C_5H_{11}$ | $C_5H_{11}$ | | | |
| 21 | H | $2,4-F_2-C_6H_3$ | $C_5H_{11}$ | $C_5H_{11}$ | | | |
| 22 | H | $4-CH_3-C_6H_4$ | $C_5H_{11}$ | $C_5H_{11}$ | | | |
| 23 | H | $4-CF_3-C_6H_4$ | $C_5H_{11}$ | $C_5H_{11}$ | | | |
| 24 | H | $3-CF_3-C_6H_4$ | $C_5H_{11}$ | $C_5H_{11}$ | | | |
| 25 | H | $C_6H_5$ | $-CH_2-C(CH_3)_2$ | $-CH_2-$ | | | |
| 26 | H | $4-Cl-C_6H_4$ | $-CH_2-C(CH_3)_2$ | $-CH_2-$ | | | |
| 27 | H | $4-Br-C_6H_4$ | $-CH_2-C(CH_3)_2$ | $-CH_2-$ | | | |
| 28 | H | $4-F-C_6H_4$ | $-CH_2-C(CH_3)_2$ | $-CH_2-$ | | | |
| 29 | H | $2-F-C_6H_4$ | $-CH_2-C(CH_3)_2$ | $-CH_2-$ | | | |
| 30 | H | $2,4-Cl_2-C_6H_3$ | $-CH_2-C(CH_3)_2$ | $-CH_2-$ | | | |
| 31 | H | $2,4-F_2-C_6H_3$ | $-CH_2-C(CH_3)_2$ | $-CH_2-$ | | | |
| 32 | H | $4-CH_3-C_6H_4$ | $-CH_2-C(CH_3)_2$ | $-CH_2-$ | | | |
| 33 | H | $4-CF_3-C_6H_4$ | $-CH_2-C(CH_3)_2$ | $-CH_2-$ | | | |
| 34 | H | $3-CF_3-C_6H_4$ | $-CH_2-C(CH_3)_2$ | $-CH_2-$ | | | |
| 35 | H | $4-Cl-C_6H_4$ | $-CH_2-CH_2$ | | | | |
| 36 | H | $4-Br-C_6H_4$ | $-CH_2-CH_2-$ | | | | |

Tabelle (Fortsetzung)

| Bsp. Nr. | $R^1$ | $R^2$ | $R^3$ | $R^4$ | Sdp. | 250 MHz $^1H$-NMR in $CDCl_3$ δ [ppm] | Ausbeute [%] |
|---|---|---|---|---|---|---|---|
| 37 | H | $4-F-C_8H_4$ | $-CH_2-CH_2-$ | | | | |
| 38 | H | $2-F-C_6H_4$ | $-CH_2-CH_2-$ | | | | |
| 39 | H | $2,4-Cl_2-C_6H_3$ | $-CH_2-CH_2-$ | | | | |
| 40 | H | $2,4-F_2-C_6H_3$ | $-CH_2-CH_2-$ | | | | |
| 41 | H | $4-CH_3-C_6H_4$ | $-CH_2-CH_2-$ | | | | |
| 42 | H | $4-CF_3-C_6H_4$ | $-CH_2-CH_2-$ | | | | |
| 43 | H | $3-CF_3-C_6H_4$ | $-CH_2-CH_2-$ | | | | |
| 44 | $C_6H_5$ | $CH_3$ | $CH_3$ | $CH_3$ | | | |
| 45 | $4-Cl-C_6H_4$ | $CH_3$ | $CH_3$ | $CH_3$ | | | |
| 46 | $4-Br-C_6H_4$ | $CH_3$ | $CH_3$ | $CH_3$ | | | |
| 47 | $4-F-C_6H_4$ | $CH_3$ | $CH_3$ | $CH_3$ | | | |
| 48 | $2-F-C_6H_4$ | $CH_3$ | $CH_3$ | $CH_3$ | | | |
| 49 | $2,4-Cl_2-C_6H_3$ | $CH_3$ | $CH_3$ | $CH_3$ | | | |
| 50 | $2,4-F_2-C_6H_3$ | $CH_3$ | $CH_3$ | $CH_3$ | | | |
| 51 | $4-CH_3-C_6H_4$ | $CH_3$ | $CH_3$ | $CH_3$ | | | |
| 52 | $4-CF_3-C_6H_4$ | $CH_3$ | $CH_3$ | $CH_3$ | | | |
| 53 | $3-CF_3-C_6H_4$ | $CH_3$ | $CH_3$ | $CH_3$ | | | |
| 54 | $CH_3$ | $C_6H_5$ | $CH_3$ | $CH_3$ | | | |
| 55 | $CH_3$ | $4Cl-C_6H_4$ | $CH_3$ | $CH_3$ | | | |
| 56 | $CH_3$ | $4Br-C_6H_4$ | $CH_3$ | $CH_3$ | | | |
| 57 | $CH_3$ | $4F-C_6H_4$ | $CH_3$ | $CH_3$ | | | |
| 58 | $CH_3$ | $2F-C_6H_4$ | $CH_3$ | $CH_3$ | | | |

Tabelle (Fortsetzung)

| Bsp. Nr. | $R^1$ | $R^2$ | $R^3$ | $R^4$ | Sdp. | 250 MHz $^1H$-NMR in $CDCl_3$ δ [ppm] | Ausbeute [%] |
|---|---|---|---|---|---|---|---|
| 59 | $CH_3$ | 2,4-$Cl_2$-$C_6H_3$ | $CH_3$ | $CH_3$ | | | |
| 60 | $CH_3$ | 2,4-$F_2$-$C_6H_3$ | $CH_3$ | $CH_3$ | | | |
| 61 | $CH_3$ | 4-$CH_3$-$C_6H_4$ | $CH_3$ | $CH_3$ | | | |
| 62 | $CH_3$ | 4-$CF_3$-$C_6H_4$ | $CH_3$ | $CH_3$ | | | |
| 63 | $CH_3$ | 3-$CF_3$-$C_6H_4$ | $CH_3$ | $CH_3$ | | | |
| 64 | $-C(OR^3)(OR^4)-(CH_2)_2$ | | $CH_3$ | $CH_3$ | | | |
| 65 | $-C(OR^3)(OR^4)-(CH_2)_2$ | | $C_5H_{11}$ | $C_5H_{11}$ | | | |
| 66 | $-C(OR^3)(OR^4)-(CH_2)_2$ | | $-CH_2-C(CH_3)_2$ | $-CH_2-$ | | | |
| 67 | $-C(OR^3)(OR^4)-(CH_2)_2$ | | $CH_2$ | $-CH_2-$ | | | |
| 68 | $-C(OR^3)(OR^4)-(CH_2)_2$ | | $CH_3$ | $CH_3$ | | | |
| 69 | $-C(OR^3)(OR^4)-(CH_2)_2$ | | $C_5H_{11}$ | $C_5H_{11}$ | | | |
| 70 | $-C(OR^3)(OR^4)-(CH_2)_2$ | | $-CH_2-C(CH_3)_2$ | $-CH_2-$ | | | |
| 71 | $-C(OR^3)(OR^4)-(CH_2)_2$ | | $-CH_2$ | $-CH_2-$ | | | |
| 72 | $-C(OR^8)(OR^9)-(CH_2)_4$ | | $CH_3$ | $CH_3$ | | | |
| 73 | $-C(OR^8)(OR^9)-(CH_2)_4$ | | $C_5H_{11}$ | $C_5H_{11}$ | | | |
| 74 | $-C(OR^8)(OR^9)-(CH_2)_4$ | | $-CH_2-C(CH_3)_2-$ | $-CH_2-$ | | | |
| 75 | $-C(OR^8)(OR^9)-(CH_2)_4$ | | $-CH_2$ | $-CH_2-$ | | | |
| 76 | $CH_3$ | $CH_3$ | $CH_3$ | $CH_3$ | | | |

## Patentansprüche

**1.** Verfahren zur Herstellung von α,α-Dialkoxyketonen der allgemeinen Formel I

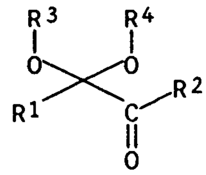

(I),

in der

$R^1$, $R^2$ unabhängig voneinander Wasserstoff, $C_1$- bis $C_{20}$-Alkyl, $C_3$- bis $C_{20}$-Cycloalkyl, $C_4$- bis $C_{30}$-Cycloalkyl-alkyl, $C_9$- bis $C_{30}$-Alkylcycloalkyl, gegebenenfalls durch $C_1$- bis $C_8$-Alkyl, $C_1$- bis $C_8$-Alkoxy, Halogen, $C_1$- bis $C_4$-Halogenalkyl, $C_1$- bis $C_4$-Halogenalkoxy, Phenyl, Phenoxy, Halogenphenyl, Halogenphenoxy und/oder Cyan substituiertes Aryl, $C_7$- bis $C_{20}$-Aralkyl oder Heterocyclus,

$R^2$ zusätzlich

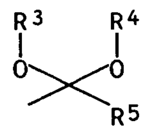

$R^3$, R4 unabhängig voneinander $C_1$- bis $C_{20}$-Alkyl, $C_3$- bis $C_{20}$-Cycloalkyl, Aryl, $C_7$- bis $C_{20}$-Arylalkyl, gemeinsam eine gegebenenfalls durch $C_1$- bis $C_4$-Alkyl substituierte $C_2$- bis $C_7$-Alkylenkette

und

$R^5$ $R^1$ oder gemeinsam mit $R^1$ eine gegebenenfalls durch $C_1$- bis $C_4$-Alkyl substituierte $C_2$- bis $C_7$-Alkylenkette

bedeuten, dadurch gekennzeichnet, daß man Ketone bzw. Aldehyde der allgemeinen Formel II

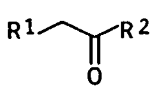

(II)

mit Nitriten der allgemeinen Formel III

$R^{3/4}$-$(NO_2)_n$ (III)

in der n = 1 oder 2 bedeutet und n = 2 ist, wenn $R^3$ und $R^4$ gemeinsam eine gegebenenfalls durch $C_1$- bis $C_4$-Alkyl substituierte $C_2$- bis $C_7$-Alkylenkette bedeutet, mit der Maßgabe, daß die Nitritgruppen endständig sind oder mit einem Gemisch aus Alkohol der allgemeinen Formel IV

$R^{3/4}$-$(OH)_n$ IV

in der n = 1 oder 2 bedeutet und n = 2 ist, wenn $R^3$ und $R^4$ gemeinsam eine gegebenenfalls durch $C_1$- bis $C_4$-Alkyl substituierte $C_2$- bis $C_7$-Alkylenkette bedeutet, mit der Maßgabe, daß die Hydroxygruppe endständig ist,

und Distickstofftrioxid in einem Molverhältnis von 2:1 in Gegenwart eines sauren Katalysators bei Temperaturen von 0 bis 170 °C, umsetzt.

Europäisches Patentamt

# EUROPÄISCHER RECHERCHENBERICHT

Nummer der Anmeldung

EP 91113709.9

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int Cl.5) |
| X<br><br><br>D | DE - A1 - 3 539 629<br>(BASF)<br>* Ansprüche 1,2 *<br>& EP-A2-0 222 217<br>-- | 1 | C 07 C 49/84<br>C 07 C 45/61 |
| A | EP - A1 - 0 122 479<br>(WACKER-CHEMIE)<br>* Anspruch 2 *<br>-- | 1 | |
| A | DE - B - 1 252 193<br>(BADISCHE ANILIN)<br>* Anspruch; Beispiele 1,2 *<br>---- | 1 | |
| | | | RECHERCHIERTE SACHGEBIETE (Int. Cl.5) |
| | | | C 07 C 45/00<br>C 07 C 49/00<br>C 07 C 41/00 |

Der vorliegende Recherchenbericht wurde für alle Patentansprüche erstellt.

| Recherchenort | Abschlußdatum der Recherche | Prüfer |
|---|---|---|
| WIEN | 05-11-1991 | REIF |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82